# EUROPEAN PATENT SPECIFICATION

(11) **EP 2 617 284 B1**
(45) Date of publication and mention of the grant of the patent: **24.06.2015**
(21) Application number: 11825257.6
(22) Date of filing: 16.09.2011
(51) Int. Cl.: A01K 67/027, C12N 15/09, G01N 33/50

(54) **MODEL ANIMAL FOR STUDYING HAIR GROWTH CYCLE**
TIERMODELL ZUM STUDIEREN EINES HAARWACHSTUMSZYKLUS
MODÈLE ANIMAL POUR L'ÉTUDE DU CYCLE DE LA POUSSE DES CHEVEUX

(30) Priority: 17.09.2010 JP 2010209689
(43) Date of publication of application: 24.07.2013
(73) Proprietor: National Institute of Advanced Industrial Science and Technology, Tokyo 100-8921 (JP)
(72) Inventor: IMAMURA Toru, Tsukuba-shi, Ibaraki 305-8566 (JP); UEKI Miho, Tsukuba-shi, Ibaraki 305-8566 (JP); ODA Yuko, Tsukuba-shi, Ibaraki 305-8566 (JP)
(74) Representative: Tuxworth, Pamela M.
(86) International application number: PCT/JP2011/071193
(87) International publication number: WO 2012/036259

(56) References cited:
- EP-A1- 2 127 674
- WO-A1-2008/102782
- WO-A1-2008/102783
- MITSUKO KAWANO ET AL: "Comprehensive analysis of FGF and FGFR expression in skin: FGF18 is highly expressed in hair follicles and capable of inducing anagen from telogen stage hair follicles", THE JOURNAL OF INVESTIGATIVE DERMATOLOGY, NATURE PUBLISHING GROUP, GB, vol. 124, 1 May 2005 (2005-05-01), pages 877-885, XP003005530, ISSN: 0022-202X, DOI: 10.1111/J.0022-202X.2005.23693.X
- KIMURA ET AL.: "Association between FGF18 signaling and resting phase of hair growth in young and adult mice", JOURNAL OF DERMATOLOGICAL SCIENCE, ELSEVIER SCIENCE PUBLISHERS, SHANNON, IE, vol. 56, no. 3, 1 December 2009 (2009-12-01), pages e1-e49, XP026789084, ISSN: 0923-1811 [retrieved on 2009-11-07]
- KOMI-KURAMOCHI, KIMURA ET AL.: "Abstracts for The 32nd Annual Meeting of the Japanese Society for Investigative Dermatology, April 18 - 20 2007, Pacifico Yokohama", JOURNAL OF DERMATOLOGICAL SCIENCE, ELSEVIER SCIENCE PUBLISHERS, SHANNON, IE, vol. 47, no. 1, 19 May 2007 (2007-05-19), pages 51-107, XP022085683, ISSN: 0923-1811, DOI: 10.1016/J.JDERMSCI.2007.03.005
- Z. LIU: "Coordination of chondrogenesis and osteogenesis by fibroblast growth factor 18", GENES & DEVELOPMENT, vol. 16, no. 7, 1 April 2002 (2002-04-01), pages 859-869, XP055104888, DOI: 10.1101/gad.965602
- EDWARD L. CHAN ET AL: "Homozygous K5Cre transgenic mice have wavy hair and accelerated malignant progression in a murine model of skin carcinogenesis", MOLECULAR CARCINOGENESIS, vol. 46, no. 1, 1 January 2007 (2007-01-01), pages 49-59, XP055104902, ISSN: 0899-1987, DOI: 10.1002/mc.20192
- TARUTANI M. ET AL.: 'Tissue-specific knockout of the mouse Pig-a gene reveals important roles for GPI-anchored proteins in skin development' PROC.NATL.ACAD.SCI.USA vol. 94, no. 14, 1997, pages 7400 - 7405, XP055075541
- KAWANO M. ET AL.: 'Comprehensive analysis of FGF and FGFR expression in skin: FGF18 is highly expressed in hair follicles and capable of inducing anagen from telogen stage hair follicles' J.INVEST.DERMATOL. vol. 124, no. 5, 2005, pages 877 - 885, XP003005530

## Description

### TECHNICAL FIELD

The present invention relates to a model animal for studying hair growth cycle in which the function of a cell growth factor is deficient or suppressed in a hair follicle-specific manner; and a screening method for drug discovery using the model animal.

### BACKGROUND ART

Recently, investigations into the molecular mechanisms of biological phenomena have progressed for improving the quality of life. With respect to hair (such as head hair, body hair and mustache/beard), an increasing number of people consider that it will become possible to promote or suppress the growth of the above-mentioned various types of hair if molecular mechanisms regulating the individual phases [growth (anagen) phase, regression (catagen) phase, resting (telogen) phase and shedding (exogen) phase] of hair growth cycle of the hair follicle (an organ producing these types of hair) and conditions of the skin have been elucidated and placed under control based on those findings; and that such promotion or suppression would contribute to the improvement of the quality of life in various social situations.

On the other hand, however, hair tonics and hair growth inhibiting agents are often developed based on ambiguous empirical rules or the like without acquisition of sufficient data using model animals. One of the major reasons for this is that rodents frequently used as model animals (such as mouse) are not suitable for screening for large number analyses performed for selecting potential drug targets from a large number of candidate substances because such rodents have physiologically a long hair growth cycle compared to their life spans. In the mouse which is a representative model animal, its dorsal skin is used for studying hair growth and hair loss. The hair growth cycle in hair follicles existing in the dorsal skin is as follows; while an anagen phase lasts for 18-19 days and a catagen phase lasts for about 2 days, a telogen phase lasts for 3-5 weeks, i.e., 21-35 days, or even longer. This means that several months or longer are required to obtain results of more than one test in the same individual. Further, since test results are greatly influenced by breeding environments such as room temperature and humidity, and individual differences are great among mice, so statistical analysis is difficult to perform in many cases. Thus, attempts to obtain highly reproducible results require enormous labor and time.

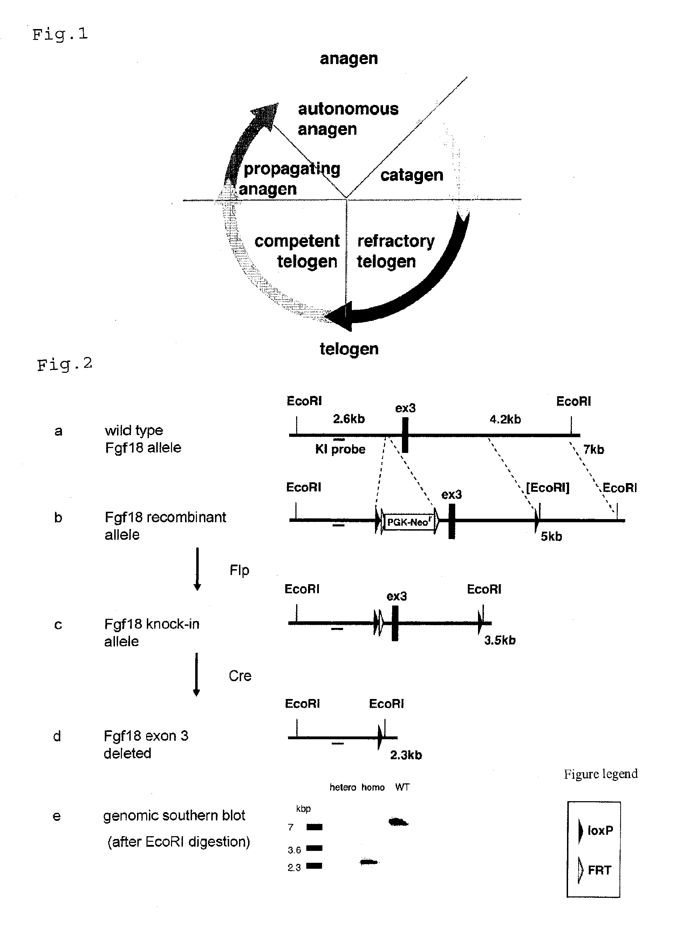

Hair growth cycle (Fig. 1) is defined by cyclic changes in the hair follicle itself (an organ which produces hair). Briefly, hair follicles repeat the three phases of anagen, catagen and telogen in this order. In the anagen phase, the lower tips of follicles reach into the subcutaneous fat layer located deeper than the skin. Simultaneously, these follicles become larger and thicker, forming long hair shafts which extend outside of the body. This is the growth of hair in appearance. In the catagen phase, as cells constituting hair follicles experience apoptosis, hair follicles retract and the lower tips thereof move into the dermis which is closer to the skin surface than the subcutaneous fat layer. In the subsequent telogen phase, it is believed that cells constituting hair follicles hardly experience growth or apoptosis but rest in a smaller state. In these catagen and telogen phases, hair growth in appearance ceases but hair does not fall. It is believed that the falling of hair occurs in an independent phase called exogen by an independent regulation. However, the exogen phase is not completely unrelated to the above-mentioned three phases; it is said that the exogen phase often occurs at the late stage of telogen or in the middle of the subsequent anagen phase.

Among the factors that define the length of each phase in hair growth cycle, cell growth factor Wnt family protein has been known as an anagen maintaining factor (Non-Patent Document No. 1), and cell growth factor FGF5 has been known as a catagen inducing factor (Non-Patent Document No. 2). However, little effort has been made to perform a detailed study of factors that are involved in maintenance and regulation of the telogen phase, factors that are involved in regulation of the exogen phase, and molecular mechanisms thereof. Therefore, together with elucidation of a regulatory factor that can shorten the duration of telogen which is the longest phase in hair growth cycle, it has been desired to supply a model animal in which hair growth cycle is sufficiently shortened by regulation of the expressin of the above-mentioned factor that it is repeated in rapid succession and occurs stably, the model animal being useful for studying hair growth cycle in hair follicles and suitable for novel drug screening pertaining to hair growth cycle in hair follicles.

### PRIOR ART LITERATURE

### Patent Documents

Patent Document No. 1: WO2008/102782
Patent Document No. 2: WO2008/102783

### Non-Patent Documents

Non-Patent Document No. 1: Shimizu, H., et al., J.Invest.Dermatol. 122, 239-245 (2004)
Non-Patent Document No. 2: Hebert, J.M., et al., Cell 78, 1017-1025 (1994)
Non-Patent Document No. 3: Zhang, X., et al., J.Biol.Chem. 281, 15694-700 (2006)
Non-Patent Document No. 4: Kawano, M., et al., J.Invest.Dermatol. 124, 877-885 (2005)
Non-Patent Document No. 5: Ohbayashi, N., et al., Genes & Dev.16, 870-879 (2002)
Non-Patent Document No. 6: Tarutani, M., et al., Proc Natl Acad Sci USA 94, 7400-7405 (1997)
Non-Patent Document No. 7: Nojima, H., Experimental Animals 55, 137-141 (2006)
Non-Patent Document No. 8: Huelsken, J., et al., Cell 105, 533-545 (2001)
Non-Patent Document No. 9: Blanpain, C., et al., Cell 118, 635-648 (2004)

### DISCLOSURE OF THE INVENTION

### PROBLEM FOR SOLUTION BY THE INVENTION

It is an object of the present invention to provide a model animal which is useful for studying the hair follicle and the skin, in particular, for studying hair growth occurring cyclically in hair follicle cells and which can be utilized in development and screening of novel drugs for hair follicle and skin diseases.

### MEANS TO SOLVE THE PROBLEM

Fibroblast growth factor 18 (FGF18) is a signaling molecule which binds to FGF receptors and causes various signal transductions downstream of the receptors (Non-Patent Document No. 3).

The present inventors previously found that FGF18 is expressed at a high level in hair follicle-containing skin, and reported that its expression level is particularly high at telogen phase in hair growth cycle (Non-Patent Document No. 4). The present inventors have also found that hair growth is remarkably inhibited when FGF18 is allowed to exit continuously under the dorsal skin of mice after compulsive induction of anagen phase in hair follicles in mouse telogen skin by hair plucking, and filed patent applications for a hair growth inhibitor comprising FGF18 and an FGF18 activating substance and a hair regrowth promoter comprising an FGF18 inhibiting substance (Patent Documents Nos. 1 and 2). Thus, the effect of the *Fgf18* gene product introduced exogenously into hair follicles exerts upon hair growth has been elucidated. However, the mechanism by which the endogenous *Fgf18* gene in hair follicle cells is expressed therein and the manner in which the gene is involved in hair growth cycle have not been well understood.

As a technique for directly elucidating the function of an endogenous gene, gene knockout technology is known. However, since FGF18 is an essential factor for organogenesis and the maintenance of life (e.g., proliferation/differentiation of chondrocytes and osteoblasts, formation of the lung, etc.), it is known that conventional systemic knockout of *Fgf18* gene hinders normal occurrence of functional morphogenesis of individuals, leading to death of the individuals at an embryonic stage or immediately after birth (Non-Patent Document No. 5).

Under these circumstances, the present inventors contemplated knocking out in a hair follicle-specific manner the *Fgf18* gene which is expressed at a high level selectively in hair follicles in the skin and skin appendages. Since the hair follicle cell is a type of keratin cells, the present inventors referred to a technology in which the gene knockout method specific to keratin-5 positive cells was applied to *Pig-a* gene involved in the synthesis of phosphatidylinositol glycan anchor (Non-Patent Document No. 6).

Specifically, a neomycin resistance gene cassette flanked by two FRT sequences was inserted upstream of exon 3 of mouse *Fgf18* gene (encoding a part of the secretion signal for FGF18 protein and a downstream region thereof). Then, a targeting vector in which the above-described neomycin cassette and exon 3 are flanked by two loxP sequences was prepared (Fig. 1). Using this targeting vector, *Fgf18* gene exon 3 allele was targeted on mouse ES cells. The resultant cells were injected into the blastocysts of C57BL/6 mice, which were then transferred into the uteri of ICR mice for development. The resultant chimeric mice were bred with C57BL/6 mice. From the resultant offspring mice, individuals harboring the introduced target gene were selected (F1). F1 individuals were bred with Flpe transgenic mouse individuals (distributed from Riken BRC; RIKEN RBRCO1837; Non-Patent Document No. 7) to thereby obtain individuals in which the neomycin resistance gene cassette flanked by FRT sequences is deleted (F2). Subsequently, these F2 mice were bred with K5-Cre transgenic mice (distributed from CARD, Kumamoto University; CARD ID323; Non-Patent Document No. 6) to thereby remove *Fgf18* gene exon 3 in a keratin-5 positive cell-specific manner. The resultant mice are "K5Cre^{tg}, Fgf18^{+/flox} mice" that are hetero-knockout mice in which one of the pair of *Fgf18* alleles is lost. "K5Cre^{tg}, Fgf18^{+/flox} mice" were bred with each other to thereby obtain "K5Cre^{tg}, Fgf18^{flox/flox} mice" that are homo-knockout mice in which both *Fgf18* alleles are completely deficient.

Like keratin-14, keratin-5 is expressed in many cells (such as epidermal cells, hair follicle cells and sebaceous cells) in the skin and skin appendages (such as hair follicle). Among them, only hair follicles express FGF18 at a high level. Therefore, the conditional knockout mouse obtained as described above can be described as a mouse which is deficient of *Fgf18* gene in a substantially hair follicle-specific manner. Accordingly, in the present specification, the homo-knockout mouse ("K5Cre^{tg}, *Fgf18*^{flox/flox} mouse"), in particular, in which both *Fgf18* alleles are completely deficient is sometimes described as a "hair follicle-specific, FGF18-deficient mouse". Since the hetero-knockout (K5Cre^{tg}, *Fgf18*^{+/flox}) mouse obtained in the process of preparing the homo-knockout mouse is deficient in one of the pair of *Fgf18* alleles in a hair follicle-specific manner, this mouse may be referred to as a "hair follicle-specific, FGF18-deficient mouse (hetero)".

In the preparation of hair follicle-specific, FGF18-deficient mice, the above-described K5-Cre transgenic mice may be replaced by K14-Cre transgenic mice in which Cre is driven by Keratin-14 gene promoter. Actually, a report has been made in which a gene expressed in hair follicles is knocked out using K14-Cre transgenic mice (Non-Patent Document No. 8). Further, it is also possible to prepare a hair follicle-specific, FGF18-deficient mouse using a transgenic mouse in which Cre is driven by the promoter of a gene encoding a keratin family protein (other than keratin-5 and keratin-14) or a keratin-binding protein, each of which is known to be expressed broadly in the hair follicle and the epidermis. Alternatively, it is also possible to prepare a hair follicle-specific, FGF18-deficient mouse using a transgenic mouse in which Cre is driven by the promoter of a gene (other than keratin genes and keratin-related genes) that is expressed in the hair follicle bulge region which expresses FGF18 at a high level. Examples of genes that are expressed in the hair follicle bulge region include, but are not limited to, a group of genes that have been reported as genes expressed selectively in the bulge region which is an epithelial stem cell niche (Non-Patent Document No. 9).

Hair follicle-specific, FGF18-deficient mice grew up healthy, were fertile and did not have apparently detectable disorders. Surprisingly, however, the present inventors have confirmed that the state of progress of their hair growth cycles is considerably different from that in wild-type mice.

Briefly, the telogen phase that lasts for 3-5 weeks or more in conventional wild-type mice lasts for only about one week in hair follicle-specific, FGF18-deficient mice; and the time period required for one cycle to complete was also shortened to about three weeks. Further, the shedding of club hairs in hair follicles was prevented, and the progress of a shedding phase was delayed. As a result, the number of hair shafts per hair follicle increased at least two-fold as a whole. Moreover, in these mice, the progress of hair growth cycles is less affected by the "hair cycle domain" structure on the body surface, and hair growth cycle proceeds in an extremely smooth manner. Further, as these hair follicle-specific, FGF18-deficient mice grow older, a stripe pattern of hair growth phases reflecting the smooth and rapid succession of hair growth cycle appears in the skin of the individuals. Therefore, with these hair follicle-specific, FGF18-deficient mice, it becomes possible to perform evaluation and screening of prophylactic or therapeutic substances for hair associated disorders and various skin diseases, and substances which promote or suppress hair regrowth or hair loss. Such evaluation and screening have been extremely difficult to perform with wild-type mice on the skin surface of the same individual with good reproducibility Besides, these hair follicle-specific, FGF18-deficient mice have a characteristic that shedding of grown-up hairs is less likely to occur. Together with rapid succession of hair growth cycle, this characteristic produces mice systemically provided with a larger number of hairs densely grown. When trimmed appropriately, these mice will have a stripe-patterned hair coat. Thus, it is possible to make use of their hairs or they may be used as ornamental or pet animals.

On the other hand, in "hair follicle-specific, FGF18-deficient mice (hetero)", it is believed that hair follicle-specific expression level of *Fgf18* gene is substantially reduced to one half. With respect to the state of progress of hair growth cycles, these mice show a nature in between wild-type mice and hair follicle-specific, FGF18-deficient mice (homotype). Briefly, the period of hair follicle telogen of these heterotype mice was 30 days, as opposed to 43 days for wild-type and 6-8 days for homotype. These hetero-knockout mice are useful in the development of model animals for studying phenomena that occur in a hair follicle-specific and FGF18 dose dependent manner, or screening methods for drug discovery using such model animals. Specifically, substances suppressing the expression of endogenous *Fgf18* gene in hair follicles or substances suppressing the activity of FGF18 protein can be screened for in the same system as using wild-type mice. At that time, compared to wild-type mice in which the highly expressed FGF18 should be reduced to below a specific level, heterotype mice with a lower expression level is capable of sensitive detection of test substances. Further, the hair cycle telogen phase is shortened to about 3/4 of the value for wild-type mice, and thus the overall hair growth cycle progresses rapidly, resulting in an advantageous faster progress of screening.

The present invention has been achieved based on the above-described findings.

From these results, it has also become clear that endogenous FGF18 in hair follicles not only extends the telogen phase of hair growth cycle but also causes club hairs within hair follicles to shed off. Thus, the present inventors have found that a substance suppressing the expression of the endogenous *Fgf18* gene in hair follicles or a substance suppressing the activity of FGF18 protein may serve as an agent for shortening the telogen phase of hair growth cycle or an agent for preventing hair loss, and filed a patent application on the same day as the present patent application was filed.

A summary of the present invention is as described below.
(1) A non-human model animal in which the expression of FGF18 is inhibited or suppressed in a hair follicle-specific manner to shorten the telogen phase of hair growth cycle, wherein at least one of a pair of fibroblast growth factor 18 (*Fgf18*) alleles has been knocked out or knocked down in a keratin 5-positive cell-specific manner.
(2) The non-human model animal of (1) above which has a larger number of hair shafts per unit area of body surface, wherein both of the *Fgf18* alleles have been knocked out or knocked down.
(3) The non-human model animal of (1) or (2) above, which is a model animal for evaluating drugs that act on hair follicles.
(4) The non-human model animal of (3) above, which is a model animal for an evaluation method in which a plurality of test substances that act on hair follicles are allowed to act simultaneously or one and the same test substance is allowed to act a plurality of times.
(5) A method of evaluating an advantageous or harmful effect of a test substance on hair follicles, comprising administering the test substance to the non-human model animal of any one of (1) to (4) above and evaluating the test substance using, as an indicator, extension of telogen phase, shortening or extension of anagen phase or acceleration of exogen phase in the non-human model animal.
(6) A method of screening for FGF18 expression suppressing substances using the non-human model animal of (1) above in which only one of the *Fgf18* alleles has been knocked out, comprising a step of observing whether or not the expression level of *Fgf18* gene is lower in the presence of an administered test substance than in the absence of the test substance.
(7) A method of screening for FGF18 activity suppressing substances using the non-human model animal of (1) above in which only one of the *Fgf18* alleles has been knocked out, comprising a step of observing whether or not the expression level of a gene located downstream of *Fgf18* gene is different in the presence of an administered test substance than in the absence of the test substance.

### EFFECT OF THE INVENTION

The present invention provides a model non-human animal for studying individual phases (for example anagen phase, catagen phase, telogen phase, exogen phase) constituting the hair growth cycle of the hair follicle (an organ that produces hair such as head hair, body hair and mustache/beard) and the states of the skin and skin appendages. In the model animal of the present invention, progress of cycles in a large number of hair follicles is little affected by the hair cycle domains on the body surface, and succession of hair growth cycles occurs rapidly. Therefore, it is possible to allow different phases of growth cycle to be expressed simultaneously on the body surface of one individual. The model animal of the present invention is also characterized by a decreased likelihood of hair shedding. Thus, the present invention provides a non-human model animal suitable for studying the hair follicle, the skin and skin appendages. At the same time, the present invention is capable of providing methods that use the non-human model animal to evaluate and screen for prophylactic or therapeutic substances for hair associated disorders and various skin diseases or substances promoting or suppressing hair regrowth or hair loss.

Although it is well known that hair loss may be caused by stress, pathogenic mechanisms of stress-induced alopecia (i.e., how stress is associated with physiological hair growth regulation) have not been elucidated sufficiently. Therefore, radical cure of alopecia is difficult; even symptomatic therapy is not sufficiently effective at present. Since hair growth cycles progress rapidly in the model animal of the present invention, this animal is useful for dealing problems that are difficult to solve by experiment with conventional model animals. It is believed that analysis of mechanisms in the model animal of the present invention will make it possible to elucidate the pathogenic mechanisms of stress-induced alopecia and like diseases. Thus, the present invention will contribute to the development of a treatment appropriately based on the mechanism of this type of alopecia and drugs for this treatment.

Further, the present invention can provide an industrially useful non-human animal or a pet animal, which utilize the above-described characteristic hair growth cycle of the non-human model animal of the present invention to make use of hair coat or use them as ornamental or pet animals.

### BRIEF DESCRIPTION OF THE DRAWINGS

[Fig. 1] Hair Growth Cycle
   In this Figure, "anagen" (growth) represents a phase in which growth of hair 9 follicles and hair shaft formation progress; "catagen" (regression) represents a phase in which hair follicles regress; and "telogen" (resting) represents a phase in which the activities of hair follicles rest. Further, this Figure shows that "anagen" is divided into "propagating anagen" and "autonomous anagen" and that "telogen" is divided into "refractory telogen" and "competent telogen". Further, it is considered that "shedding phase (exogen)" in which a hair shaft apparently falls out is not directly linked to catagen or telogen but occurs as an independent phase during the telogen subsequent to the anagen where the relevant hair shaft was completed or during the subsequent anagen phase.
[Fig. 2] Schematic View of *Fgf18* Gene Knockout
   a: Structure around exon 3 of mouse *Fgf18* gene
      In this Figure, the thick black line indicates exon 3, and the horizontal line shows intron structure. "EcoRI" indicates the position of restriction enzyme EcoRI site.
   b: Illustration of the structure of targeting vector and genomic homologous recombination
      In this Figure, "KI probe" represents a DNA probe used for judgments of recombination and genotypes in Southern blotting; and its homologous position is indicated with a bold line. The boxed PGK-Neo^{r} represents a neomycin resistance gene cassette; black triangle represents loxP sequence; and gray triangle represents FRT sequence.
   c: This Figure shows the sequence of b above from which the neomycin resistance gene cassette flanked by FRT sequences has been removed with Flp recombinase.
   d: This Figure shows the sequence of c above from which exon 3 of *Fgf18* gene flanked by loxP sequences from has been removed with Cre recombinase.
   e: One example of the results of Southern blot analysis
   Analysis results for hetero-, homo- and wild-type are shown.

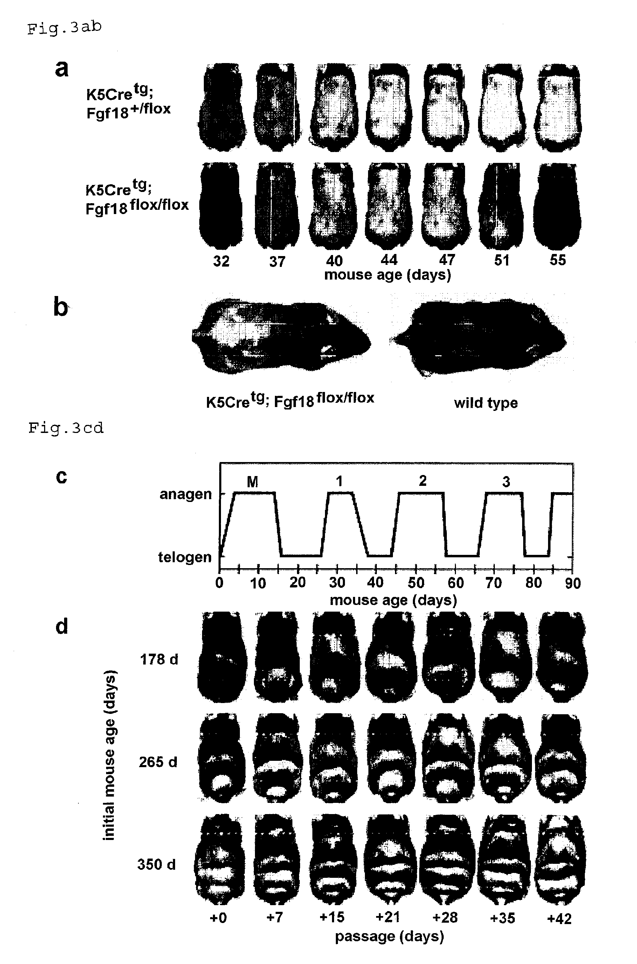

[Fig. 3a, b] Rapid Succession of Hair Growth Cycles in Hair Follicle-Specific, FGF18-Deficient Mouse (K5Cre^{tg}; *Fgf18*^{flox/flox}; homo-knockout mouse)
   a: Transition of hair growth cycles in the dorsal skin was observed chronologically on one representative individual for each of hair follicle-specific, FGF18-deficient mouse (homo-knockout mouse; lower panel) and the control hetero-knockout mouse (K5Cre^{tg}, *Fgf18*^{+/flox}; upper panel) at early ages. Each photograph was taken after the hair was trimmed short with a hair clipper. Skin colors are reflecting the phases of hair growth cycle.
   b: One example of hair growth pattern in the dorsal skin is shown on one representative adult individual for each of hair follicle-specific, FGF18-deficient mouse (homo-knockout mouse; left) and the control wild-type mouse (C57BL/6; right).
[Fig. 3c, d] Rapid Succession of Hair Growth Cycles in Hair Follicle-Specific, FGF18-Deficient Mouse (K5Cre^{tg}; *Fgf18*^{flox/flox}; homo-knockout mouse) (2)
   c: Changes in hair growth cycle in the dorsal skin of hair follicle-specific, FGF18-deficient mouse (homo-knockout mouse) (Y axis) was plotted against mouse age in days (X axis).
   d: Transition of hair growth cycles in the dorsal skin of aged, hair follicle-specific, FGF18-deficient mouse (homo-knockout mouse) was observed approximately every one week. Each photograph reflects the hair growth in one week before the day of observation.
[Fig. 3e] Comparison of Telogen Duration in Hair Follicle-Specific, *Fgf18* Gene-Deficient Mice (homozygous and heterozygous) and Wild-Type Mouse.
[Fig. 4] Illustration of Hair Cycle Domains
   This Figure shows the hair cycle domains existing in the dorsal skin of mouse. Hair cycle domains exist in the dorsal skin of mouse like partitions delineated by vertical and horizontal lines as shown in this Figure. It is known empirically that the regulation of hair growth differs from domain to another. It should be noted that the numbers and positions of vertical and horizontal lines in this Figure are only for illustration. Actually, various patterns exist.

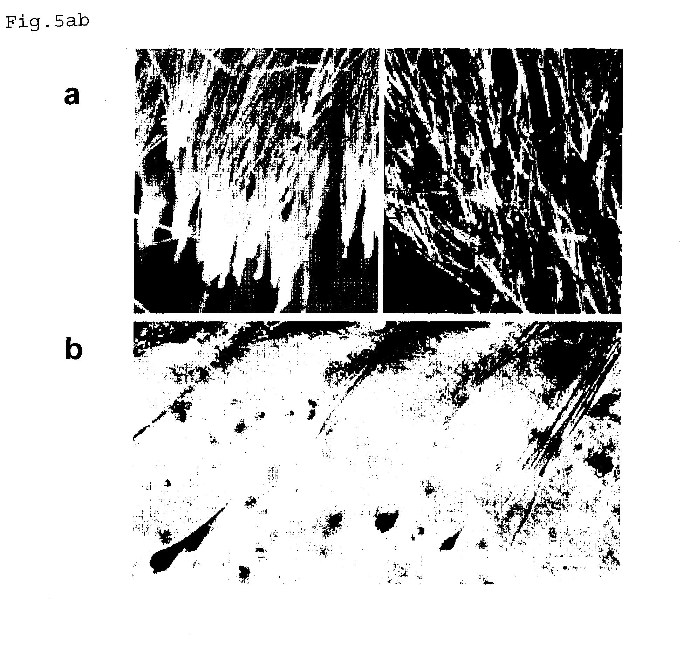

[Fig. 5a, b] Incomplete Shedding of Club Hairs in Aged, Hair Follicle-Specific, FGF18-Deficient Mouse (Homo-Knockout Mouse)
   a: Left panel: the state of club hairs plucked with fingers from the back of aged, hair follicle-specific, FGF18-deficient mouse (homo-knockout mouse). Right panel: the state of club hairs plucked from a control hetero-knockout mouse of the same age.
   b: The state of hair follicles around anagen phase hair follicles. Bar represents 200 µm.

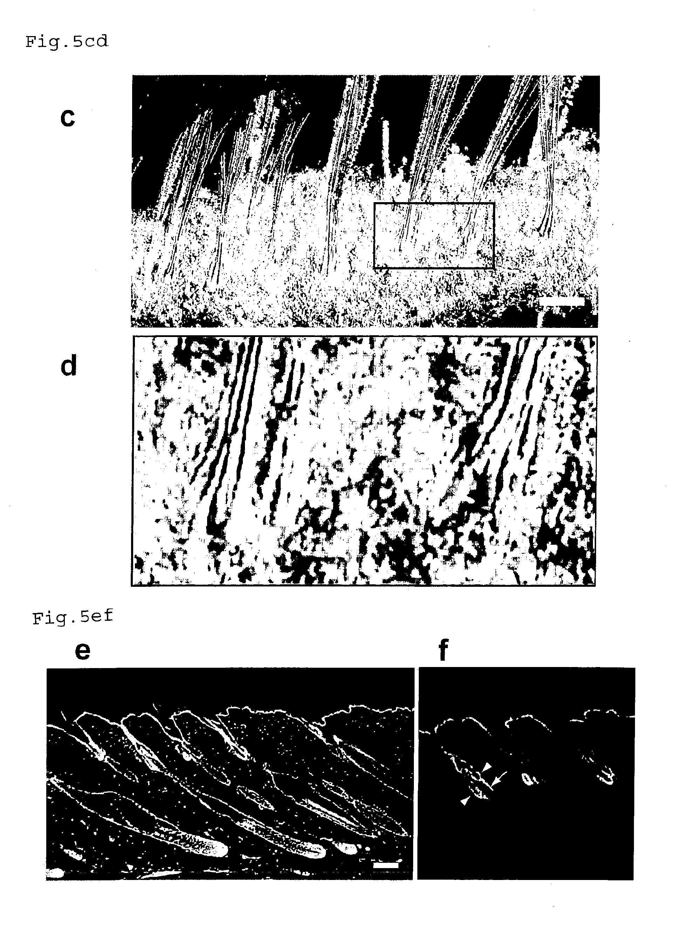

[Fig. 5c, d] Incomplete Shedding of Club Hairs in Aged, Hair Follicle-Specific, FGF18-Deficient Mouse
   c: The state of hair follicles around telogen phase hair follicles. Bar represents 200 µm.
   d: An enlarged view of the boxed portion in panel c.
[Fig. 5e, f] Incomplete Shedding of Club Hairs in Aged, Hair Follicle-Specific, FGF18-Deficient Mouse
   e: Photograph of an immunostained skin section containing anagen hair follicles. Double staining against keratin-15 and PCNA antigen was performed. Hair follicles of club hairs alongside the bulge regions of PCNA-positive anagen hair follicles are observed.
   f: Only keratin-15 signals in a partial field of the above panel e are shown. Keratin-15 is an antigen characteristic of skin stem cells, and it is seen that the bulge region of anagen hair follicles and the bag-like structure enclosing club hair each form a keratin-15 positive and continuous structure.

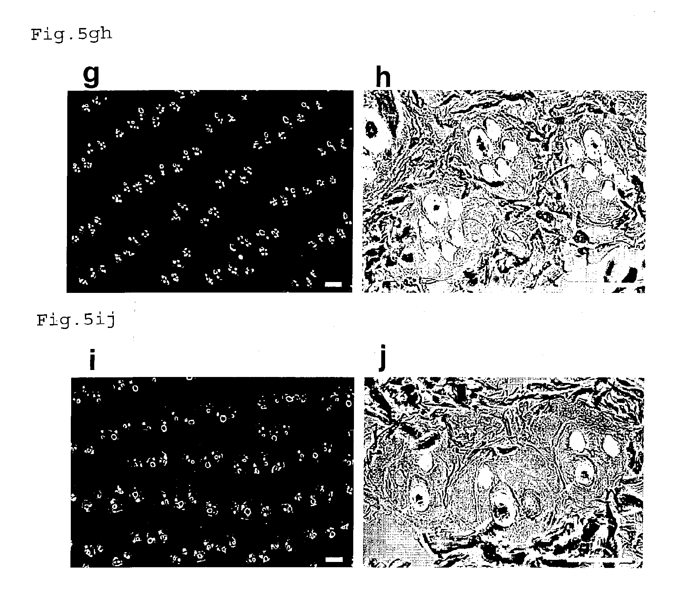

[Fig. 5g, h] Incomplete Shedding of Club Hairs in Aged, Hair Follicle-Specific, FGF18-Deficient Mouse
   g: A fluorescence image of a cross section of a sample around anagen phase hair follicles from the dorsal skin of aged, hair follicle-specific, FGF18-deficient mouse (homo-knockout mouse). The section is stained with thioflavin T, a fluorescent dye that stains hair shaft proteins. Bar represents 100 µm.
   h: An enlarged image of the same sample as used in panel e. The image is merged with a corresponding phase contrast image. Bar represents 100 µm. Arrows indicate melanin-containing hair shafts growing in anagen hair follicles; others are club hairs.
[Fig. 5i, j] Incomplete Shedding of Club Hairs in Aged, Hair Follicle-Specific, FGF18-Deficient Mouse
   i: A control, fluorescence image of a cross section of a sample around anagen hair follicles from the dorsal skin of hetero-knockout mouse of the same age as the mice in panels e and f The section is stained with thioflavin T, a fluorescent dye that stains hair shaft proteins. Bar represents 200 µm.
   j: An enlarged image of the same sample as used in panel g. The image is merged with a corresponding phase contrast image. Bar represents 100 µm. Arrows indicate melanin-containing hair shafts growing in anagen hair follicles; others are club hairs.

### BEST MODES FOR CARRYING OUT THE INVENTION

### 1. "Hair Follicle-Specific, FGF18-Deficient Non-Human Animal" of the Present Invention

The expression "hair follicle-specific, FGF18-deficient non-human animal" or "hair follicle-specific, FGF18-knockout non-human animal" used in the present specification refers to a tissue-specific, knockout, non-human animal in which the expression of *Fgf18* gene (as inherently and selectively expressed in hair follicles in the skin and skin appendages, e.g., hair follicle) is deficient because *Fgf18* gene has been replaced with a corresponding incomplete gene in a keratin-5 positive cell-specific manner. The "expression of *Fgf18* gene is deficient" means that the expression of *Fgf18* gene does not occur at all or if it is expressed, the function of normal *Fgf18* gene product can not be displayed.

A representative "hair follicle-specific, FGF18-deficient non-human animal" is a tissue-specific, knockout, non-human animal as prepared by using the Cre-loxP method to delete exon 3 of *Fgf18* gene in the genome sequence in a keratin-5 positive cell-specific manner. As a result of deletion of exon 3, part of the secretion signal for FGF18 protein and a downstream region thereof will not be expressed, whereupon the biological activity of FGF18 protein is lost.

Further, a transgenic (knockdown) animal may be obtained by integrating an shRNA into a retrovirus or adenovirus vector and then introducing the resultant vector into an animal such that it is delivered to its skin and hair follicles. In that case, the function of *Fgf18* gene will be suppressed in a hair follicle cell-specific manner in the skin of the animal. (Hereinafter, such an animal is also referred to as hair follicle-specific, *Fgf18* gene-suppressed non-human animal).

The species of the model animal of the present invention is not particularly limited. For example, bovine, sheep, goat, porcine, equine, canine, feline, rabbit, chicken, rat or mouse may be used. Among these, rodents are preferable, and mouse is particularly preferable. In Examples of the present invention, Flpe mouse distributed from RIKEN BRC is used to delete a neomycin cassette from a targeting vector, and also used therein is K5-Cre mouse distributed from CARD, Kumamoto University, having a keratin-5 positive cell-specific promoter linked to Cre. These mice may be prepared by applying the techniques disclosed in Non-Patent Documents Nos. 8 and 9, respectively, to a conventional mouse line C57BL/6.

### 2. Method of Preparation of "Hair Follicle-Specific, FGF18-Deficient Non-Human Animal" of the Present Invention

The "hair follicle-specific, FGF18-deficient non-human animal" of the present invention is prepared as described below.
(1) A non-human animal that is deficient of *Fgf18* gene in a keratin-5 positive cell-specific manner may be prepared by the procedures described below using a well-known gene targeting method (if necessary, see Transgenic Mouse: Methods and Protocols; Series; Methods in Molecular Biology, Volume 209; Pub. Date: Aug-20-2002; 9. Conditional Knockout Mice, By: Ralf Kuhn, Frieder Schwenk, Pages 159-185; DOI: 10.1385/1-59259-340-2: 159; Springer) consulting the method disclosed in Non-Patent Document No. 6.

### (a) Acquisition of the Target Region of Fgf18 Gene

First, a DNA fragment containing a region of *Fgf18* gene to be deleted (e.g., a region containing exon 3) is obtained from the genomic library. Any region of *Fgf18* gene may be deleted as long as no *Fgf18* gene product is yielded or the function of FGF18 expression product can be lost. Since exon 3 of *Fgf18* gene encodes part of the secretion signal of FGF18 protein and a downstream region thereof, the biological activity of FGF18 protein can effectively be lost by deleting exon 3. Thus, this region was selected as a target region in Examples of the present invention.

Since the nucleotide sequence of a genomic region containing exon 3 of mouse-derived *Fgf18* gene may be obtained from a public database such as GenBank (Entrez Gene; Gene ID: 14172, updated on 21-Jul-2010; Official Symbol: *Fgf18* provided by MGI; Official Full Name: fibroblast growth factor 18 provided by MGI; Primary source: MGI: 1277980), PCR primers may be designed so that a region containing exon 3 is flanked by the primers. Then, a clone corresponding to the target region may be obtained from an appropriate mouse genomic library (e.g., Bacterial Artificial Chromosome: BAC Clone Library). In other non-human animals such as bovine, sheep, goat, swine, equine, chicken and rat, the same operations are carried out on a gene homologous to *Fgf18* gene. For example, the nucleotide sequence of a rat gene homologous to *Fgf18* may be obtained from a database such as GenBank in the same manner (Gene ID: 29369).

### (a) Construction of Targeting Vector

In the present invention, a targeting vector for deleting exon 3 of *Fgf18* gene in a keratin-5 positive cell-specific manner is constructed using Cre-loxP system (R. Kuhn et al., Science, 269, 1427-1429, 1995). From the above step (a) to this step (b), the vector construction may be outsourced to a company such as PhoenixBio Co., Ltd. by identifying a target region to be knocked out of the genome sequence of a known gene. In the present invention, the targeting vector was outsourced to PhoenixBio Co., Ltd.

Specifically, a neomycin resistance gene cassette flanked by two FRT sequences is inserted upstream of exon 3 of *Fgf18* gene; then, a targeting vector in which this neomycin cassette and exon 3 are flanked by two loxP sequences is prepared (Fig. 1b). "loxP sequence" is a 34 bp sequence found in the genome of bacteriophage P1. It has a structure that an asymmetric 8 bp sequence at the center is flanked by two sets of a 13 bp symmetric sequence. The DNA sequence flanked by two loxP sequences can be deleted with a site-specific recombinase Cre protein that is capable of causing recombination between the loxP sequences in the DNA. Likewise, "FRT sequence" is a sequence which is the target of a site-specific recombinase Flp (Flp Recombinase Target); a DNA sequence flanked by two FRT sequences is removed by Flp.

### (b) Homologous Recombination in ES Cells

A targeting vector in which *Fgf18* gene has been modified so that its function becomes deficient is introduced into ES cells according to a known method with necessary modifications. With a generally known recombination technique (for example, Nature, Vol. 350, No. 6315, p.243. 1991), the function-deficient *Fgf18* gene is substituted for the corresponding wild-type gene on the genome of ES cells to thereby prepare mutant clones. As a method for introducing a target vector into ES cells, a known method such as electroporation, the liposome method, the calcium phosphate method, or the DEAE-dextran method may be used. However, considering the homologous recombination efficiency of the transgene, use of electroporation is preferable. ES cells have also been established in non-human animals other than mouse (e.g., rat) and easily available. Alternatively, iPS cells, somatic stem cells or fertilized eggs may be used in place ofES cells.

Further, whether or not homologous recombination has occurred in recombinant ES cells is preferably determined by introducing a drug resistance factor such as neomycin into the targeting vector in advance and performing screening in a selective medium. Further, those cells in which homologous recombination has occurred correctly can be selected by Southern hybridization or PCR assay.

### (d) Preparation of Individuals (F1) Harboring the Target Gene

The thus prepared, homologously recombined ES cell clones are introduced into blastcysts of fertilized eggs or 8-cell stage embryos of a non-human animal. Then, the ES cell embryos are transferred into the uterus of a non-human animal as a pseudopregnant foster mother, which is allowed to deliver offspring, whereby a chimeric non-human animal can be prepared. In order to confirm that the ES cells have been introduced into the germline, various phenotypes such as coat color may be used as indicators. Alternatively, the confirmation can be made by Southern blot analysis or PCR assay after extraction of DNA from a part of the body (e.g., the tail tip). The resultant chimeric non-human animal is bred with a wild-type non-human animal. From the resultant offspring, those individuals harboring the introduced target gene (F1) are selected.

Alternatively, a chimeric individual consisting of embryonic stem cell clones and normal cells may be prepared by a technique such as injection chimera or aggregation chimera method. By breeding this chimeric individual with a normal individual, a hetero individual can be obtained which has an *Fgf18* allele on the chromosomes of cells of its entire body and a corresponding allele prepared by introducing a desired mutation into the *Fgf18* allele. Thus, the allelic type of this individual is hetero.

### (e) Removal of Target Vector-Derived Drug Resistance Gene Cassette (F2)

To prepare the target vector in (b) above, a drug resistance gene (such as neomycin resistance gene) cassette flanked by two FRT sequences was inserted. Now, this cassette is quickly removed from the genome sequence.

In Examples of the present invention, F1 individuals were bred with Flpe transgenic mice distributed from RIKEN BRC (RIKEN RBRC01834; Non-Patent Document No. 8) to thereby obtain F2 individuals in which the neomycin resistance gene cassette flanked by FTR sequences has been deleted (Fig. 1c). However, the method of removing such a cassette is not limited to this technique. For information, the above-described Flpe transgenic mouse is a genetically modified mouse expressing a site-specific recombinase Flp systemically. When a DNA region flanked by FRT sequences that are targets of recombinase Flp exists in chromosomes of this mouse, the DNA region is removed systemically.

### (f) Keratin-5 Positive Cell-Specific Deletion of Fgf18 Gene Function

Subsequently, the F2 individuals described above are bred with non-human animal individuals expressing Cre recombinase in a keratin-5 positive cell-specific manner and the Cre/loxP system is driven to delete *Fgf18* gene function in a keratin-5 positive cell-specific manner.

In Examples of the present invention, the F2 individuals described above were bred with K5-Cre transgenic mice (distributed from CARD, Kumamoto University; Non-Patent Document No. 9). For information, the above-described K5-Cre mouse is a genetically modified mouse which expresses a site-specific recombinase Cre only in those cells where keratin-5 protein is expressed. When a DNA region flanked by loxP sequences that are targets of Cre exists in chromosomes of this mouse, the DNA region is removed only in keratin-5 positive tissues such as hair follicles. In other tissues, the gene targeted by loxP sequences is not removed. By breeding F2 individuals with K5-Cre mice, a mouse is obtained which heterozygously has chromosomes in which *Fgf18* exon 3 region has been deleted in a keratin-5 positive cell-specific manner (Fig. 1d). By breeding these hetero-deficient mouse individuals with each other, it is possible to obtain the "hair follicle-specific, FGF18-deficient mouse" of the present invention which is homo-deficient. Deletion of *Fgf18* exon 3 region in hair follicles may be confirmed by examining the genotype of Fgf18 gene and the expression of K5-Cre by PCR on samples from the resultant mouse pups containing the skin (e.g., a part of the tail).

Preparation of other non-human animals may be performed by the same procedures as described above.

### 3. Method of Preparation of Hair Follicle-Specific, Fgf18 Gene-suppressed Non-Human Animal

A transgenic (knockdown) animal which overexpresses an siRNA against *Fgf18* gene in a hair follicle-specific manner can be obtained by linking an shRNA of *Fgf18* gene to a keratin-5 positive cell-specific promoter, integrating the shRNA into a retrovirus or adenovirus vector, and introducing the resultant vector into a non-human animal such that it is delivered to the animal's skin and hair follicles.

### 4. Characteristics of Hair Follicle-Specific, Fgf18 Gene-Deficient Non-Human Animal

Hereinbelow, hair follicle-specific, *Fgf18* gene-deficient mouse which is the most representative as a hair follicle-specific, *Fgf18* gene-deficient non-human animal will be mainly described. However, it is needless to say that the non-human animal of the present invention is not limited to mouse.

### (4-1) Overall characteristics

In mammals such as mouse, *Fgf18* gene is expressed in bones, cardiac muscle, embryonic lung and other tissues, and is believed to play an important role in organogenesis and the maintenance of life. However, among the skin and skin appendages such as hair follicle, it is only the hair follicles that expresses *Fgf18* at a high level. Further, considering the entire body of an animal, keratin-5 gene is expressed at a high level in only a very small number of tissues such as bone, tongue and bronchi, aside from the skin. Therefore, in the conditional knockout mouse obtained as described above, the only part that may be described as having substantially lost the physiological function of *Fgf18* gene in comparison with wild-type mouse is hair follicles. For this reason, individuals of the hair follicle-specific, *Fgf18* gene-deficient non-human animal of the present invention are in very healthy conditions and can be used as a model animal for studying the hair follicle and the skin. This model animal can be used to examine the effect of test substances upon hair growth cycle, to evaluate their applicability as prophylactics or therapeutics for hair loss or hair regrowth or a hair growth promoter, or to perform screening based on the evaluation. Thus, this model animal is extremely useful.

Characteristics observed specifically in the hair follicle-specific, *Fgf18* gene-deficient non-human animal of the present invention are as described below.
(1) Since the telogen of in hair growth cycle is shortened to 1/3-1/5, the progress of hair growth cycles is rapid.
(2) Hair growth cycles in the dorsal skin proceed systematically to form zonal pattern. The proceeding speed is highest at the anterior region near the neck and lowest at the posterior region near the tail. Since the proceeding speed increases systematically, in a zonal pattern, a plurality of transverse stripes are formed upon repeated trimming. The number of these stripes is proportional to aging.
(3) Although the number of hair follicle cells does not increase, the speed of hair shedding from hair follicles is reduced. As a result, the number of hair shafts in hair follicles becomes larger, and the number of hair shafts in the total skin surface becomes more than twice the value for wild-type animal.

### (4-2) Characteristically Rapid Progress of Hair Growth Cycles

In the model animal of the present invention, hair growth occurs in by far rapid cycles compared to wild-type animal. In particular, telogen in hair growth cycle is short (Fig. 3c). Taking mouse as an example (Fig. 3c), it has been shown in the model animal of the present invention that telogen ceases in about one week to be followed by subsequent anagen. On the other hand, in wild-type mouse, it is well known that telogen starting at about 48 days of age in Fig. 3c usually lasts for 3-5 weeks. Briefly, the model animal of the present invention is a non-human animal model in which telogen in hair growth cycle is shortened to 1/2-1/10, preferably 1/3-1/5, of the value for wild-type mouse. As a result, the hair growth cycle itself of the non-human model animal of the present invention is shortened to 1/2-1/10, preferably 1/3-1/5. In the hair follicle-specific, FGF18-deficient mouse of the present invention, the time period required for one cycle to complete is shortened to about 3 weeks, whereas the value for wild-type mouse is about 6-8 weeks or even longer.

Briefly, it is believed that inhibition of the expression of endogenous *Fgf18* gene in hair follicles resulted in a shortened telogen of hair growth cycle and, at the same time, accelerated the speed of hair growth cycle itself.

### (4-3) Inherent Phenotypes of Hair Growth Cycle Phases

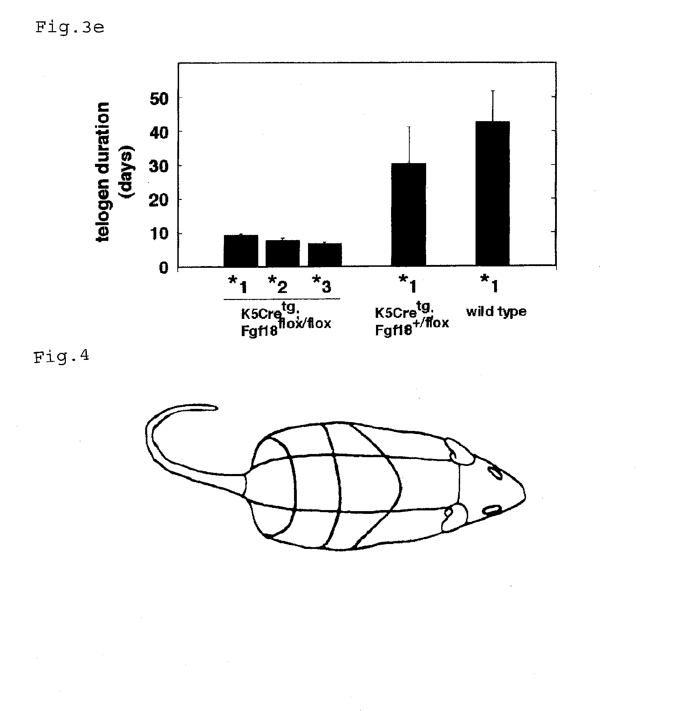

A tendency that hair growth cycle in the dorsal skin of mouse proceeds most rapidly at the anterior region near the neck and most slowly at the posterior region of the tail has been already known. However, hair growth cycle starts at a plurality of positions in the dorsal skin. When the hair in the dorsal skin is trimmed in wild-type mouse, hair growth occurs at random throughout the dorsal skin, as seen in the right panel of Fig. 3b. In order to explain this phenomenon, the existence of a regulatory system called "hair cycle domains" (divisions in blocks) which determines hair growth cycle was generally assumed in the dorsal skin of mouse (Fig. 4). Hair cycle domains exist in the dorsal skin of mouse; they seem to be partitioned with vertical and horizontal lines as shown in Fig. 4. It is empirically known that regulation of hair cycle differs from one domain to another. However, the numbers and positions of vertical and horizontal lines in Fig. 4 are provided only for illustration. Actually, there are various patterns.

On the other hand, the hair follicle-specific, *Fgf18* gene-deficient mouse of the present invention does not show such hair cycle domains. It is very likely that a factor governing the regulatory system which is divided into blocks to determine hair growth cycle has been lost. The tendency inherent in the progress speed of hair growth cycles (i.e., hair growth cycle in the dorsal skin of mouse proceeds most rapidly at the anterior region near the neck and most slowly at the posterior region of the tail) is expressed as it is. Consequently, as mouse grows older, hair follicles in individual phases are seen to be aligned in an orderly way, just like stripes of a zebra. Briefly, it is believed that if the expression of endogenous *Fgf* gene in hair follicles is inhibited, borderlines between hair cycle domains running at right angles to the body axis of a non-human animal are lost.

Therefore, hair follicle states having a plurality of phases in the same growth cycle appear simultaneously in the dorsal skin of this model mouse. By utilizing this characteristic, the effects of a test substance upon individual phases can be examined at a time. Further, since hair growth cycles proceed rapidly, this model mouse is most appropriate for judging the effect on hair growth of slow-acting test substances which must be administered over a plurality of hair growth cycles until their effect develops. Further, application to a non-human model animal in which the above-described stripe pattern itself can be enjoyed will give birth to a unique pet animal.

### (4-4) Characteristically Large Number of Hair Shafts in Hair Follicles

In the hair follicle-specific, *Fgf18* gene-deficient mouse of the present invention, the number of follicles per unit area of the skin is not increased but the beginning of shedding phase is delayed despite the accelerated progress of hair growth cycles, and the shedding of hairs as products of the preceding cycle is less likely to occur. In hair follicles, there are a large number of club hairs that stay alongside anagen hair shafts growing in anagen hair follicles and which are yet to shed off. Briefly, it is believed that inhibition of the expression of endogenous *Fgf* gene in hair follicles delayed the start time of shedding phase to thereby prevent the shedding of club hairs.

As a result, the number of hair shafts per hair follicle becomes larger than in wild-type mouse. A non-human model animal covered with body hairs 1.5 to 6 times, preferably at least twice, as many as the body hairs on wild-type mouse in number can be obtained. Thus, a novel pet animal whose body surface is densely covered with a thick coat of body hairs can be provided. Further, in the case of non-human animals whose body hairs are to be used industrially, the value of their industrial utility increases since the volume of body hairs that can be shorn is increased.

### 2. Effects of Fgf18 Gene in Hair Follicle Cells and FGF18 Protein as Gene Product

From comparative analysis of phenotypes of *Fgf18* gene knockout mouse and wild-type mouse, *Fgf18* gene in hair follicle cells and FGF18 protein as gene product are presumed to have the following effects.
(1) FGF18 has a function of maintaining the telogen phase of hair follicles. Briefly, it is believed that endogenous FGF18 in hair follicles is a regulator which extends telogen phase in hair growth cycle.
(2) FGF18 has a function of causing club hairs to shed from the skin. Briefly, it is believed that endogenous FGF18 in hair follicles is a regulator which starts hair shedding phase that has not been elucidated to date.
(3) FGF18 has a function of determining borderlines between hair cycle domains sharing a direction running at right angles (tentatively regarded as Y axis) to the body axis of a non-human animal (tentatively regarded as X axis). Briefly, it is believed that endogenous FGF18 is one of the regulators which determine hair cycle domains in the skin surface.

### 3. Methods of Evaluation and Screening Using the Model Animal of the Present Invention

The non-human animal of the present invention may be used in the screening for evaluating test substances using indicators such as extension of telogen phase, shortening or extension of anagen phase or acceleration of shedding phase in the model animal. The model animal of the present invention is particularly useful in the screening for those substances which are effective for extending short telogen phase or in the screening for evaluating drugs more than once that act on a plurality of hair follicle cells.

The test substance to be subjected to the evaluation method of the present invention or a screening method based on this method may be any known compound or novel compound. For example, nucleic acids, saccharides (glycoconjugates), lipids, proteins, peptides, organic low molecular weight compounds, compound libraries constructed with combinatorial chemistry technique, random peptide libraries constructed by solid-phase synthesis or the phage display method, and natural components derived from microorganisms, animals/plants or marine organisms may be enumerated.

In the present invention, an animal at about 6 weeks of age or older is used as a model animal for screening for those substances which are effective in extending short telogen phase. Further, as a model animal for screening for therapeutics for hair associated disorders, it is effective to use a mouse, for example, at 40 weeks of age or older. At this age, a plurality of stripes formed by hair growth phases appear simultaneously in the dorsal skin at a probability of 90% or more.

The evaluation of test substances of the present invention is performed as described below. As an example, evaluation of telogen extension effect using mouse as a model animal will be given below. A test substance of an appropriate concentration is administered to hair follicle-specific, *Fgf18* gene-deficient mice under breeding by application to the dorsal skin or subcutaneous injection. Alternatively, the test substance may be mixed with drinking water or feed and then taken *ad libitum* for an appropriate period, or administered to mice at an appropriate dose by intraperitoneal injection. It is preferable to set appropriate conditions as to, for example, concentration, dose, frequency of administration, and period of administration. After a specific period (of administration), the dorsal skin of an individual mouse is observed to judge the telogen extending effect of the test substance. If necessary, the mouse is dissected, and its skin tissues are analyzed by, for example, the tissue staining method.

In a preferred embodiment, wild-type mouse or heterotype hair follicle-specific, *Fgf18* gene-deficient mouse may be used as a control, analyzed simultaneously under the same conditions, and compared for the results of analysis for judgment. However, with wild-type mouse, it is difficult to evaluate telogen extension correctly, so judgment is made as to whether or not the results obtained from wild-type mouse is contradictory to the results obtained from hair follicle-specific, *Fgf18* gene-deficient mouse.

### 4. Screening for Hair Follicle-Specific, Fgf18 Gene Expression Suppressing Substance or FGF18 Activity Suppressing Substance

Hereinbelow, screening using heterotype hair follicle-specific, *Fgf18* gene-deficient mouse will be described. Screening using other heterotype hair follicle-specific, *Fgf18* gene-deficient non-human animal may be performed in the same manner.

The heterotype hair follicle-specific, *Fgf18* gene-deficient mouse of the present invention has hair follicle-specific *Fgf18* gene expression activity, though the expression level is reduced considerably. Therefore, this mouse may be used in screening for hair follicle-specific,*Fgf18* gene expression suppressing substances or FGF18 activity suppressing substances in the same manner as wild-type mouse is used. The resultant hair follicle-specifically *Fgf18* gene expression inhibiting substances or FGF18 activity inhibiting substances are candidates for hair cycle telogen phase shortening agents, hair loss preventing agents or fur conditioning agents.

Further, in heterotype hair follicle-specific, *Fgf18* gene-deficient mouse, its telogen phase is shortened to about 3/4 of the value for wild-type mice, and thus the overall hair growth cycle progresses rapidly, resulting in an advantageous faster progress of screening. Further, the level of expression of *Fgf18* gene in hair follicles is considerably low as compared to wild-type mouse, so there is also an advantage in that the *Fgf18* gene expression suppressing effect of test substances can be observed with high sensitivity.

Specifically, screening for hair follicle-specific, *Fgf18* gene expression suppressing substances or FGF18 activity suppressing substances may be performed as described below.

First, a test substance is administered to a heterotype hair follicle-specific, *Fgf18* gene-deficient mouse. The test substance is not particularly limited. For example, plant extracts, peptides, proteins, nonpeptidic compounds, low molecular weight compounds, synthetic compounds, fermentation products, cell extracts, and animal tissue extracts may be enumerated. These substances may be either novel or known substances. When a test substance is to be administered to an experimental animal, especially in the case where the test substance is a protein, a gene encoding the test substance may be introduced into FGF receptor expressing cells.

Subsequently, the expression of *Fgf18* gene in the experimental animal is monitored. The expression of *Fgf18* gene in the experimental animal may be monitored, for example, by analysis with a conventional method such as ELISA using FGF18 antibody or by analyzing the mRNA level of *Fgf18* gene in the experimental animal as through quantitative reverse transcription PCR or Northern blotting.

When the results from any of these analyses show that the expression level of *Fgf18* gene in the experimental animal is smaller than in the absence of the test substance, the test substance can be judged as potentially having the function of hair growth promotion or hair regrowth, namely as a candidate for hair growth promoter or hair regrowth promoter. Specifically, when the level of the mRNA of *Fgf18* gene in the presence of the test substance is reduced to a value which is 0.8 times or less, preferably 0.7 times or less, more preferably 0.5 times or less, compared to the value for the case of the absence of the test substance, the test substance can positively be described as an FGF18 expression suppressing substance. Since the expression levels of the mRNA of FGF18 in cultured keratinocytes, cultured dermal cells and cultured dermal papilla cells vary widely depending on culture conditions and cell types, the expression level may be determined individually by, for example, the method described above and screening may be performed with reference to a guide figure which is a level reduction to 0.8 times or less.

When the activity of FGF18 is suppressed in the experimental animal by administration of a test substance, the expression levels of genes located downstream of *Fgf18* in the FGF18 signaling pathway (hereinafter, called *Fgf18* downstream genes) should be affected. Therefore, by monitoring the expression levels of *Fgf18* downstream genes, it is possible to judge as to whether or not the FGF18 activity has been suppressed by the test substance.

Briefly, in the same manner as in the above-described *Fgf18* gene expression monitoring method, if the expression levels of *Fgf18* downstream genes in the experimental animal are different in the presence of a test substance than in the absence of the test substance, it is possible to judge that FGF18 activity has been suppressed by the presence of the test substance and that the test substance potentially has the function of hair growth promotion or hair regrowth. Specifically, an *Fgf18* downstream gene suppressed by FGF18 in telogen hair follicles and their vicinity is selected as a target of monitoring. If the mRNA level of the *Fgf18* downstream gene whose expression is suppressed by FG18 activity is increased to a value which is 1.5 times or more, preferably 2 times or more, more preferably 3 times or more, compared to the value for the absence of the test substance, the test substance is judged as an FGF18 activity suppressing substance.

The FGF18 expression suppressing substance or FGF18 activity suppressing substance thus screened through the above-described steps may be used either alone or in combination as a hair growth telogen shortening agent, a hair loss preventing agent, or as a fur conditioning agent.

Hereinbelow, the present invention will be described more specifically with reference to Examples. However, the present invention is not limited or restricted by these Examples.

### EXAMPLES

Unless otherwise noted, known methods described in Molecular Cloning (Molecular Cloning: A laboratory Manual published by Sambrook and Russell) were used as genetic engineering techniques.

### (Example 1) Preparation of Hair Follicle-Specifically F2f18 Gene-Deficient (Knockout) Mouse

BAC clone containing mouse *Fgf18* gene was obtained from 129 mouse BAC clone library. Using this clone, a targeting vector for deleting exon 3 of *Fgf18* gene was constructed; in this vector, loxP-FRT[PGK-Neo^{r}]FRT sequence was introduced upstream of exon 3 of *Fgf18* gene and loxP sequence was introduced downstream thereof (Fig. 2). The preparation of this targeting vector was outsourced to PhenixBio as "a targeting vector for deleting exon 3 of mouse *Fgf18* gene".

This vector was introduced into 129 mouse-derived ES cells. From the resultant cell population, cells in which *Efg18* gene had been replaced with the above-described sequence (to be referred to as "targeted") were selected and cloned. One of the resultant ES cell clones was used for preparation of a transgenic mouse. These ES cells were injected into blastocysts of C57BL/6 mice (dark brown coat; Japan SLC), and then the blastocysts were introduced into the uteri of pseudopregnant female ICR mice (white coat; Japan SLC). After birth, chimeric offspring mice were identified by coat color. These operations were carried out for 63 blastocysts to thereby obtain 16 chimeric pups. These chimeric mice were reared until sexually matured and then bred with C57BL/6 mice to yield 35 pups. Analysis of the genome of these pups revealed that 15 individuals were harboring the targeted *Fgf18* gene (these mice are designated F1).

Subsequently, for removing the [PGK-Neo^{r}] cassette, F1 mice were bred with Flpe transgenic mice to yield 67 pups. Analysis of the *Fgf18* alleles of these pups confirmed that 12 individuals harbored the sequence of interest (loxP-FRT-Exon3-loxP sequence from the introduced loxP-FRT[PGK-Neo^{r}]FRT-Exon3-loxP) (F2).

The Flpe transgenic mice used herein were obtained from RIKEN BRC (RIKEN RBRC01834; Non-Patent Document No. 8).

Subsequently, for the purpose of removing exon 3 of *Fgf18* gene in a hair follicle-specific manner, F2 mice were further bred with K5-Cre transgenic mice. The resultant pups were brought to weaning at 4 weeks of age, and skin containing tail samples were cut off. Then, genotype of *Fgf18* gene and expression of Cre were confirmed on these samples. Among 37 individuals successfully weaned, six KSCre^{tg};*Fgf18*^{+/flox} mice (one female and five males) were obtained. Mating between K5Cretg;*Fgf18*^{+/flox} mice resulted in 29 offspring mice. Analysis of genotypes of these mice revealed that one individual was KSCre^{tg};*Fgf18*^{flox/flox} (hereinafterreferred to as *Fgf18* homo-deficient mouse). A great number of *Fgf18* homo-deficient mice could be obtained by mating a KSCretg;*Fgf18*^{+/flox} mouse colony in the same manner

Hereinafter, this *Fgf18* homo-deficient mouse is designated "hair follicle-specifically *Fgf18* gene-deficient animal".

The K5Cre transgenic mice used herein were obtained from CARD, Kyushu University (CARD ID323; Non-Patent Document No. 9).

The existence of a DNA deficient of exon 3 of *Fgf18* gene in the skin of these mice was confirmed by Southern blotting. Such examples are shown below.

Further, the genotypes of the offspring mice produced from the mating between KSCretg;*Fgf18*^{+/flox} mice showed a normal Mendelian segregation pattern.

### (Example 2) Shortening of Telogen Phase and Rapid Succession of Hair Growth Cycles in Hair Follicle-Specific, Fgf18 Gene-Deficient (Knockout) Mouse

### (2-1) Observation of Hair Growth Cycles in Hair Follicle-Specific, Fgf18 Gene-Deficient (Knockout) Mouse

Hair follicle-specific, *Fgf18* gene-deficient mice survived and grew; they were fertile and seen to be healthy in appearance. By trimming the body hair growing in the dorsal skin, characteristic waves of hair growth cycles appeared. Fig. 3a shows photographs of a representative individual (homo-knockout mouse) [K5Cre^{tg};*Fgf18*^{flox/flox}] of hair follicle-specific, *Fgf18* gene-deficient mice and, as a control, a representative individual (hetero-knockout mouse) [KSCre^{tg};*Fgf18*^{+/flox}] of control group mice in which only one of a pair of *Ffg18* alleles was knocked out; these photographs were taken at 32 days of age and thereafter. Prior to the photographing, their body hair was trimmed short with a hair clipper so that the color tones of their body surfaces could be easily seen. At 32 days of age, the dorsal skin in both mice was in the first physiological anagen, presenting black color. At 37 days of age, a region near the neck turned into a pinkish color, indicating that it entered telogen. At 40 days of age, the entire dorsal skin was in telogen. In the case of homo-knockout mouse, bluish coloring of the skin appearing at 47 days indicated that the mouse entered the subsequent anagen (Fig. 3a). By contrast, telogen continued for a much longer period in the control hetero-knockout mouse (Fig. 3a).

Hair growth cycles in the dorsal skin of hair follicle-specific, *Fgf18* gene-deficient mouse (68 days of age) progressed smoothly from the anterior position (near the neck) to the posterior position (near the tail). It can be seen that in addition to hair follicle morphogenesis (a cycle expressed by "M" in Fig. 3c), two hair growth cycles (1 and 2 in Fig. 3c) had already occurred to enter the third cycle (3 in Fig. 3c) (Fig. 3b, left panel). By contrast, in wild-type mice (89 days of age), the onset time and location of the subsequent anagen varied among individuals and even in the dorsal skin of one individual, different phases were presented on account of "hair growth domains" (Fig 3b, right panel and Fig. 4). Therefore, in hair follicle-specific, *Fgf18* gene-deficient mouse, transition of individual phases in general hair growth cycles can be shown as a function of mouse age as in Fig. 2a (see Fig. 3c).

Here, phases in hair growth cycle in a dorsal skin region near the neck (a portion surrounded by a dotted line in Fig. 3b) are judged by skin color and hair growth history in the preceding week, and presented as either anagen or telogen for simplicity. As it turned out, telogen lasted for only about one week and each hair growth cycle as a whole including telogen was about three weeks in the hair follicle-specific, *Fgf18* gene-deficient mouse. By contrast, telogen usually lasts for 3-5 weeks or even longer in wild-type mouse.

Fig. 3d shows three representative individuals of aged hair follicle-specific, *Fgf18* gene-deficient mice. These mice were reared for one week after trimming of their hair in the dorsal skin. Then, the mice with the hair grown during that one week were photographed. A characteristic phenotype of these aged hair follicle-specific, *Fgf18* gene-deficient mice was such that hair follicles in the same growth cycle phase were aligned so that they formed a stripe in appearance. As a further characteristic, the number of stripes was found to increase with the mouse's age (days). In aged mice, a time period required for one hair growth cycle to complete was about three weeks in the anterior position (Fig. 3d) but about four weeks in the posterior position (Fig. 3d). Therefore, it is understood that this difference in the length of growth cycle between the anterior position and the posterior position shortens the distance between stripes in aged hair follicle-specific, *Fgf18* gene-deficient mice.

### (2-2) Shortening of Telogen in Hair Follicle-Specific, Fgf18 Gene-Deficient Mouse

In hair follicle-specific, *Fgf18* gene-deficient (homo-knockout) mouse (K5Cre^{tg};*Fgf18*^{fllox/flox}), hetero-knockout mouse with only one *Fgf18* allele (KSCre^{tg};*Fgf18*^{+/flox}) and a control wild-type mouse with both *Fgf18* alleles, hair follicle anagen and telogen were judged and the duration of telogen was analyzed in the same manner as in Fig. 3c. The results are shown. Three or more individuals per group were analyzed. Mean and standard deviation were calculated and graphed. In Fig. 3e, *1 indicates the duration of telogen starting at about 37 days of age; *2 and *3 indicate the durations of telogen phases starting at about 58 days and 78 days of age, respectively Since telogen lasts for a long period of time in each of hetero-knockout mouse group and wild-type mouse group, *2 and *3 are difficult to analyze. Thus, only telogen duration of * 1 is shown.

These results clearly reveal that telogen is shortened in hair follicle-specific, *Fgf18* gene-deficient (homo-knockout) mouse.

### (Example 3) Incomplete Physiological Hair Shedding in Hair Follicle-Specific, Fgf18 Gene-Deficient Mouse

Hairs growing in telogen hair follicles are hairs that have been completed in the preceding hair growth cycle and are designated "club hairs". Generally, in mouse, club hairs growing in telogen hair follicles can be picked up with gloved fingers and gently plucked. It was found that the thus plucked hairs are in the form of bundles in hair follicle-specific, *Fgf18* gene-deficient (homo-knockout) mouse (Fig. 5a, left panel).

Such bundles were hardly observed in the control hetero-knockout mouse (Fig. 5a, right panel). Examination of more than 200 plucked hairs from each group showed that the hair length was almost equal in homo-knockout mouse (6.40+/-0.53mm) and hetero-knockout mouse (6.38+/-0.65mm). Further, the distribution of each hair type (i.e., guard, awl and zigzag hairs) did not differ between the two groups. From these results, it was strongly suggested that the anagen of hair growth cycle was proceeding normally in hair follicle-specific, *Fgf18* gene-deficient mouse.

The number of all hair follicles in a 3.7 mm² region of dorsal skin characterized by anagen of hair cycle and the number of hair shafts present in the follicles were counted in aged hair follicle-specific, *Fgf18* gene-deficient mouse (homo-knockout mouse) and control hetero-knockout mouse (Table 1).

**Table 1. Number of Hair Shafts Retained by Hair Follicle Cells**

| State of Hair Shafts in Hair Follicles | | Number of Hair Follicles | |
|---|---|---|---|
| Number of Anagen Hair Shafts per Hair Follicle | Number of Club Hair Shafts per Hair Follicle | Hair Follicle-Specific, Fgf18 Knockout Mouse *K5Cre^{tg};Fgt18*^{*flox*/*flox*} (43 week-old male) | Control Mouse *K5Cre^{tg};Fgt18*^{+/*flox*} (43 week-old male) |
| 1 | 0 | 1 | 34 |
| 1 | 1 | 3 | 73 |
| 1 | 2 | 28 | 46 |
| 1 | 3 | 41 | 4 |
| 1 | 4 | 61 | 0 |
| 1 | 5 | 20 | 0 |
| 1 | 6 | 1 | 0 |
| 0 | 1 | 1 | 0 |
| 0 | 2 | 4 | 4 |
| 0 | 3 | 2 | 0 |
| 0 | 4 | 2 | 0 |
| | | | |
| Total Number of Hair Follicles | | 164 | 161 |
| Total Number of Hair Shafts | | 710 | 342 |

These results show that, although the total number of hair follicles in hair follicle-specific, *Fgf18* gene-deficient mouse is almost equal to that in control group, the greater number of hair shafts as retained in each hair follicle provided a total number of hair shafts that was a little more than twice the value for the control group.

Further, aged hair follicle-specific, *Fgf18* gene-deficient mouse (homo-knockout mouse) and control hetero-knockout mouse were examined as enlarged for the state of hair follicles in the dorsal skin. The results revealed that a great number of club hairs stayed alongside anagen hair shafts growing in anagen hair follicles and were yet to shed off (Fig. 5c). Further, full focus imaging of the skin confirmed that a bag-like structure enclosing the club hairs that did not shed off was positioned alongside the bulge region of growing hair shafts as if they were united to the latter. Further, cross sections of hair follicles of aged hair follicle-specifically *Fgf18* gene-deficient mouse were prepared, stained with thioblavin T (yellow color) and DAPI (red color), and examined. As a result, it was shown that in homo-knockout mouse, each follicle retained both a melanin-rich anagen hair shaft and mostly three to six melanin-free club hairs (Fig. 5f). By contrast, in control hetero-knockout mouse, the number of club hairs retained in an anagen hair follicle was mostly one to two. The distribution was as shown in Table 1. These observations suggest that the mechanism for normal shedding of club hairs (shedding phase) was incomplete in hair follicle-specific, *Fgf18* gene-deficient mouse (homo-knockout mouse). Even if this is the case, the shedding of hair shafts, though incomplete, would have occurred to some extent because the number of residual hair shafts per hair follicle was smaller than the number of hair growth cycles that was considered to have been completed (10 to 12 cycles).

## Claims

1. A non-human model animal in which the expression of FGF18 is inhibited or suppressed in a hair follicle-specific manner to shorten the telogen phase of hair growth cycle, wherein at least one of a pair of fibroblast growth factor 18 (*Fgf18*) alleles has been knocked out or knocked down in a keratin 5-positive cell-specific manner.

2. The non-human model animal of claim 1 which has a larger number of hair shafts per unit area of body surface, wherein both of the *Fgf18* alleles have been knocked out or knocked down.

3. The non-human model animal of claim 1 or 2, which is a model animal for evaluating drugs that act on hair follicles.

4. The non-human model animal of claim 3, which is a model animal for an evaluation method in which a plurality of test substances that act on hair follicles are allowed to act simultaneously or one and the same test substance is allowed to act a plurality of times.

5. A method of evaluating an advantageous or harmful effect of a test substance on hair follicles, comprising administering the test substance to the non-human model animal of any one of claims 1 to 4 and evaluating the test substance using, as an indicator, extension of telogen phase, shortening or extension of anagen phase or acceleration of exogen phase in the non-human model animal.

6. A method of screening for FGF18 expression suppressing substances using the non-human model animal of claim 1 in which only one of the *Fgf18* alleles has been knocked out, comprising a step of observing whether or not the expression level of *Fgf18* gene is lower in the presence of an administered test substance than in the absence of the test substance.

7. A method of screening for FGF18 activity suppressing substances using the non-human model animal of claim 1 in which only one of the *Fgf18* alleles has been knocked out, comprising a step of observing whether or not the expression level of a gene located downstream of *Fgf18* gene is different in the presence of an administered test substance than in the absence of the test substance.

## Patentansprüche

1. Nichtmenschliches Tiermodell, in dem die Expression von FGF18 haarfollikelspezifisch gehemmt oder unterdrückt ist, um die telogene Phase des Haarwachstumscyclus zu verkürzen, wobei mindestens einer eines Paares von Fibroblastenwachstumsfaktor 18 (*Fgf18*)-Allelen keratik-5-positv-zellenspezifsch aus- oder heruntergeschaltet wurde.

2. Nichtmenschliches Tiermodell nach Anspruch 1, das eine größere Anzahl von Haarschäften pro Flächeneinheit der Körperoberfläche aufweist, wobei beide der *Fgf18-*Allele aus- oder heruntergeschaltet wurden.

3. Nichtmenschliches Tiermodell nach Anspruch 1 oder 2, welches ein Tiermodell zur Bewertung von Arzneimitteln ist, die auf Haarfollikel wirken.

4. Nichtmenschliches Tiermodell nach Anspruch 3, welches ein Tiermodell für ein Bewertungsverfahren ist, in dem eine Vielzahl von Testsubstanzen, die auf Haarfollikel wirken, gleichzeitig oder einzeln wirken gelassen wird, und die gleiche Testsubstanz viele Male wirken gelassen wird.

5. Verfahren zur Bewertung einer vorteilhaften oder schädlichen Wirkung einer Testsubstanz auf Haarfollikel, umfassend das Verabreichen der Testsubstanz an das nichtmenschliche Tiermodell nach einem der Ansprüche 1 bis 4 und das Bewerten der Testsubstanz unter Verwendung, als ein Indikator, der Extension der telogenen Phase, der Verkürzung oder der Extension der anagenen Phase oder der Beschleunigung der exogenen Phase in dem nichtmenschlichen Tiermodell.

6. Verfahren zum Screening auf FGF18-expressionsunterdrückende Substanzen unter Verwendung des nichtmenschlichen Tiermodells nach Anspruch 1, in dem nur eines der (*Fgf18*)-Allele ausgeschaltet wurde, umfassend einen Schritt des Feststellens, ob das *Fgf18*-Gen-Expressionsniveau in Gegenwart einer verabreichten Testsubstanz geringer ist als in Abwesenheit der Testsubstanz oder nicht.

7. Verfahren zum Screening auf FGF18-aktivitätsunterdrückende Substanzen unter Verwendung des nichtmenschlichen Tiermodells nach Anspruch 1, in dem nur eines der (*Fgf18*)-Allele ausgeschaltet wurde, umfassend einen Schritt des Feststellens, ob das Expressionsniveau eines stromabwärts von dem *Fgf18*-Gen gelegenen Gens in Gegenwart einer verabreichten Testsubstanz verschieden ist von der Abwesenheit der Testsubstanz oder nicht.

## Revendications

1. Modèle animal non humain dans lequel l'expression du FGF18 est inhibée ou supprimée de manière spécifique à un follicule pileux afin de raccourcir la phase télogène du cycle de la pousse des cheveux, où au moins l'un d'une paire d'allèles du facteur de croissance des fibroblastes 18 (*Fgf18*) a été invalidé ou inactivé de manière spécifique aux cellules positives pour la kératine 5.

2. Modèle animal non humain selon la revendication 1, qui possède un plus grand nombre de tiges pilaires par unité de surface corporelle, dans lequel les deux allèles de *Fgf18* ont été invalidés ou inactivés.

3. Modèle animal non humain selon la revendication 1 ou 2, qui est un modèle animal pour évaluer des médicaments qui agissent sur les follicules pileux.

4. Modèle animal non humain selon la revendication 3, qui est un modèle animal pour un procédé d'évaluation dans lequel on permet à une pluralité de substances de test qui agissent sur des follicules pileux d'agir simultanément ou on permet à une seule et même substance de test d'agir plusieurs fois.

5. Procédé d'évaluation d'un effet avantageux ou nocif d'une substance de test sur des follicules pileux, comprenant l'administration de la substance de test au modèle animal non humain selon l'une quelconque des revendications 1 à 4, et l'évaluation de la substance de test en utilisant, en tant qu'indicateur, un prolongement de la phase télogène, un raccourcissement ou un prolongement de la phase anagène ou une accélération de la phase exogène chez le modèle animal non humain.

6. Procédé de criblage de substances supprimant l'expression du FGF18 en utilisant le modèle animal non humain selon la revendication 1 dans lequel un seul des allèles de *Fgf18* a été invalidé, comprenant une étape qui consiste à observer si le taux d'expression du gène *Fgf18* est plus faible ou non en présence d'une substance de test administrée plutôt qu'en l'absence de la substance de test.

7. Procédé de criblage de substances supprimant l'activité du FGF18 en utilisant le modèle animal non humain selon la revendication 1 dans lequel un seul des allèles de *Fgf18* a été invalidé, comprenant une étape qui consiste à observer si le taux d'expression d'un gène localisé en aval du gène *Fgf18* est différent ou non en présence d'une substance de test administrée plutôt qu'en l'absence de la substance de test.
